# EUROPÄISCHE PATENTANMELDUNG

(11) **EP 1 717 253 A1**
(43) Veröffentlichungstag der Anmeldung: **02.11.2006**
(21) Anmeldenummer: 05103574.9
(22) Anmeldetag: 29.04.2005
(51) Int. Cl.: C08G 14/06, C07C 215/34, C08L 63/00, C08L 75/00

(54) **Mannichbase auf Resorcin-Basis**

(71) Anmelder: Sika Technology AG, 6340 Baar (CH)
(72) Erfinder: Gerber, Ulrich, 8142, Uitikon-Waldegg (CH)
(74) Vertreter: Isler, Jörg

(57) **Zusammenfassung**

Die vorliegende Erfindung betrifft Mannichbasen, welche aus Resorcin, Formaldehyd, sowie Triethylentetramin und/oder Tetraethylenpentamin herstellbar sind. Weiterhin wird ein Verfahren zu deren Herstellung und deren Verwendung als Härter für Amin-reaktive Verbindungen offenbart. Besonders geeignet haben sich die Mannichbasen als Härterkomponente in Klebstoffen.

## Beschreibung

### Technisches Gebiet

Die vorliegende Erfindung betrifft das Gebiet der Herstellung und Verwendung von Mannichbasen.

### Stand der Technik

Die Klasse der Mannichbasen ist schon seit langem bekannt und wurde bereits in Härterkomponenten von reaktiven Systemen eingesetzt. Zur Herstellung werden Phenole eingesetzt. Phenol (Hydroxybenzol) als Ausgangsmaterial hat jedoch den grossen Nachteil, dass die daraus hergestellten Mannichbasen noch Anteile von unreagiertem Phenol enthalten. Aufgrund der Toxizität von Phenol sind auf Phenol basierende Mannichbasen für viele Marktbereiche nicht einsetzbar. Deshalb sind grosse Bestrebungen getätigt worden, Phenol-freie Mannichbasen herzustellen. So wurden beispielsweise Mannichbasen auf Basis von Nonylphenol oder p-tert.- Butylphenol oder Cardanol entwickelt und kommerzialisiert.

Mannichbasen werden vor allem als Beschleuniger für Epoxidharze oder als Härter für Epoxidharze und Polyurethane eingesetzt. WO 00/15687 beschreibt beispielsweise einen Mannichbasen-Beschleuniger, welcher durch Transaminierung einer Mannichbase mit einem Amin hergestellt wird.

Die Verfahren zur Herstellung bekannter Mannichbasen sind sehr aufwändig und schwierig zu Führen, insbesondere dann, wenn die Ausbildung von hochmolekularen Kondensationsprodukten möglichst verhindert werden soll. So offenbart beispielsweise EP-A-1 475 411 ein zweistufiges Herstellverfahren zur Herstellung von Mannichbasen basierend auf m-Kresol oder 3,5-Xylenol und Polyaminen, bei dem vorzugsweise ein tertiäres Amin verwendet wird. Ebenso ein zweistufiges Herstellverfahren von Mannichbasen wird von EP-A-1 475 412 offenbart, wobei diese aus Phenolen wie m-Kresol, 3,5-Xylenol oder Resorcin mit Polyaminen vorzugsweise unter Verwendung von tertiären Aminen gewonnen werden. Derartige zweistufige Verfahren sind jedoch mit zusätzlichen Aufwand verbunden und verteuern die Produktion der Mannichbasen.

### Darstellung der Erfindung

Aufgabe der vorliegenden Erfindung ist es daher, neue Mannichbasen sowie das Verfahren zu deren Herstellung zur Verfügung zu stellen, welche sich frei von Phenol sind und sich durch ein vereinfachtes Verfahren herstellen lassen.

Überraschenderweise hat sich gezeigt, dass durch eine spezifische Auswahl von im Stand der Technik bekannten Polyamin und phenolischen Verbindungen Mannichbasen gemäss des Anspruches 1 hergestellt werden können, welche diese Aufgabe lösen können. Diese Mannichbasen sind aus billigen, einfach zugänglichen Rohstoffen über eine einfache Herstellung herstellbar. Sie zeichnen sich durch ein exzellentes Härtungsverhalten, insbesondere bei tiefen Temperaturen, mit Amin-reaktiven Verbindungen aus.

Weitere Aspekte der Erfindung sind in den weiteren Hauptansprüchen beschrieben. Weitere vorteilhafte Ausgestaltungen der Erfindung ergeben sich aus den Unteransprüchen.

### Wege zur Ausführung der Erfindung

Die vorliegende Erfindung betrifft Mannichbasen, welche herstellbar sind aus Resorcin, Formaldehyd und/oder Triethylentetramin und/oder Tetraethylenpentamin.

Resorcin (CAS-Nr. [108-46-3]) ist kommerziell breit in unterschiedlichen Reinheiten erhältlich. Resorcin zeichnet sich gegenüber den anderen Dihydroxybenzol-Isomeren Brenzcatechin und Hydrochinon unter anderem durch eine geringere Toxizität (Deutsche Wassergefährdungsklasse WGK 1 gegenüber 2 oder 3 oder Schweizer Giftklasse 3 gegenüber 2) aus. Es hat besonders gezeigt, dass Resorcin gegenüber diesen, wie auch anderen Phenolen wie beispielsweise Phenol, die verschiedenen Isomeren von Kresol oder Xylenol sich überraschenderweise äusserst gut für die Herstellung der Mannichbasen eignet.

Formaldehyd kann in dem Fachmann üblicherweise bekannten Formen direkt oder aus formaldehydabspaltenden Verbindungen zur Anwendung kommen. Bevorzugt ist Formaldehyd in Form als para-Formaldehyd oder als Formalin-Lösung. Besonders bevorzugt ist Formalin-Lösung.

Weiterhin wird für die Herstellung der erfindungsgemässen Mannichbasen Triethylentetramin und/oder Tetraethylenpentamin verwendet. Sowohl Triethylentetramin (TETA) (CAS Nr. [112-24-3])(3,6-Diazaoctan-1,8-diamin) als auch Tetraethylenpentamin (TEPA) (CAS Nr. [112-57-2]) (3,6,9-Triazaundecan-1,11-diamin) sind kommerziell breit erhältlich und sind sehr kostengünstig. Sie werden insbesondere in technischer Qualität angeboten und benutzt. Derartige technische Qualität wird bevorzugt. Es ist dem Fachmann bekannt, dass TETA und TEPA in derartig technischer Qualität nicht eine reine, einheitlich chemische Substanz ist. Insbesondere enthalten sie aufgrund des Verfahrens zu deren Herstellung weitere Substanzen und Isomeren. Als hauptsächlich gebildete derartige Substanzen und Isomeren gelten bei TETA:
- N,N'-bis-(2-Aminoethyl)piperazin (BisAEP oder DiAEP)(CAS-Nr. [6531-38-0])
- Piperazinoethylethylendiamin (PEEDA) (CAS-Nr. [24028-46-4])
- Tris-(2-Aminoethyl)amin (NTEA oder NTE) (CAS-Nr. [4097-89-6]) beziehungsweise bei TEPA:
   - 4-Aminoethyltriethylenetetramin (AETETA) (CAS-Nr. [31295-46-2])
   - Aminoethylpiperazinoethylethylendiamin (AEPEEDA) (CAS-Nr. [31295-54-2])
   - Piperazinoethyldiethylentriamine (PEDETA) (CAS-Nr. [31295-49-5]).

Weitere Verbindungen und Isomeren können zu geringem Masse gebildet werden, jedoch ist deren Summe neben den oben explizit erwähnten Verbindungen mengenmässig unter 3 Gew.-% bezogen auf das Gewicht des technischen TETA oder TEPA. Es kann eine Mischung von TETA und TEPA eingesetzt werden oder lediglich TETA oder lediglich TEPA.

Beide Polyamine TETA und TEPA weisen ein hohes N/C-Verhältnis auf und demzufolge kann eine Menge Aminogruppen mittels einem kleinem Molekül und demzufolge mit einer geringen Menge eingebracht werden.

Die aus Resorcin, Formaldehyd und TETA und/oder TEPA hergestellte Mannichbase weist vorzugsweise eine Aminzahl von zwischen 800 und 1100 mg/g KOH, insbesondere im Bereich zwischen 900 und 1000 mg/g KOH, bevorzugt im Bereich zwischen 950 und 1000 mg/g KOH, auf. Es lassen sich Mannichbasen herstellen, welche keine messbaren Mengen an nicht umgesetzten Resorcin mehr aufweisen.
die Aminzahl.

Ein weiterer Aspekt der vorliegenden Erfindung betrifft die Herstellung der beschriebenen Mannichbase.

Hierzu wird Resorcin, Triethylentetramin und/oder Tetraethylenpentamin mit Formaldehyd bei einer Temperatur von unter 25°C zur Reaktion gebracht. Insbesondere wird zu einer Vormischung von Resorcin und Triethylentetramin und/oder Tetraethylenpentamin Formaldehyd unter Rühren und Kühlen, auf eine Temperatur unter 25 °C, insbesondere unter 15°C, zugegeben. Die Zugabe erfolgt vorzugsweise portionenweise, insbesondere als Zutropfen. Die Resorcin/[TETA und/oder TEPA]- Vormischung wird vorzugsweise vorgängig auf eine Temperatur von etwa 80°C aufgeheizt um das Resorcin zu lösen und wieder abgekühlt, bevor das Formaldehyd zugegeben wird. Es hat sich gezeigt, dass es von Vorteil ist, wenn die Vormischung weiterhin ein Lösungsmittel, insbesondere ein Alkohol, bevorzugt Methanol, umfasst, um das Resorcin besser zu lösen und um die Viskosität zu senken, und welches vor der Reaktion mit Formaldehyd zugegeben wird. Insbesondere vorteilhaft wird das Lösungsmittel bereits ganz am Anfang, d.h. bei der Herstellung der Vormischung, verwendet. Nachdem das Formaldehyd zugetropft worden ist, wird vorzugsweise das Reaktionsgemisch auf eine Temperatur erhöht und insbesondere auf etwa 95 °C und gleichzeitig ein leichtes Vakuum von typischerweise 0.6 bis 0.9 bar angesetzt. Hierbei wird das allenfalls durchs Formaldehyd eingebrachte Wasser, sowie das gebildete Wasser sowie das gegebenenfalls verwendete Lösungsmittel abdestilliert. Das verwendete Lösungsmittel ist demzufolge vorteilhaft so auszuwählen, dass es bei dieser Temperatur und Druck leicht abzudestillieren ist.

Als besonders vorteilhaft hat sich gezeigt, dass die Mannichbasen auch ohne die Anwesenheit von zusätzlichen tertiären Aminen, welche nicht bereits in technischem Triethylentetramin und/oder technischem Tetraethylenpentamin vorhanden sind, hergestellt werden können.

Die molaren Verhältnisse von Resorcin zu Formaldehyd zur Summe von TETA und TEPA sind insbesondere 1 : 1.5-2.5 : 2.5-3.5. Als besonders geeignet hat sich ein derartiges Verhältnis von 1 zu etwa 2 zu etwa 3 erwiesen.

Die derart gebildete Mannichbase ist vorzugsweise frei von nicht umgesetztem Resorcin, d.h. dass keine messbaren Mengen von Resorcin in der Mannichbase vorhanden sind, und weist insbesondere eine Aminzahl von zwischen 800 und 1100 mg/g KOH, insbesondere im Bereich zwischen 900 und 1000 mg/g KOH, bevorzugt im Bereich zwischen 950 und 1000 mg/g KOH, auf.

Die Mannichbase kann als solche oder in einer Zusammensetzung eingesetzt werden.

Die Mannichbasen eignen sich insbesondere als Härter für eine Amin-reaktive Substanz, welche mindestens zwei Amin-reaktive funktionelle Gruppen aufweist. Als derartige Amin-reaktive funktionelle Gruppen kommen insbesondere Glycidylether- und/oder Isocyanat-Gruppen in Betracht.

In einer Ausführungsform ist die Amin-reaktive Substanz, welche mindestens zwei Amin-reaktive funktionelle Gruppen aufweist, ein Diglycidylether. Insbesondere ist es ein Diglycidylether von Bisphenol-A, Bisphenol-F oder Bisphenol-A/F, ist. Besonders bevorzugt ist ein derartiger Diglycidylether ein sogenanntes Flüssigharz, insbesondere wie sie unter dem Handelsnamen Araldite® GY 250, Araldite® PY 304, Araldite® GY 282 (Huntsman) oder D.E.R 331 (Dow) auf dem Markt erhältlich sind.

In einer weiteren Ausführungsform ist die Amin-reaktive Substanz, welche mindestens zwei Amin-reaktive funktionelle Gruppen aufweist, ein Polyisocyanat oder ein mindestens zwei Isocyanat-Gruppen aufweisendes Prepolymer ist. Als Polyisocyanat sind insbesondere 1,6-Hexamethylendiisocyanat (HDI), 2,2,4- und 2,4,4-Trimethyl-1,6-hexamethylendiisocyanat (TMDI), 1-Isocyanato-3,3,5-trimethyl-5-isocyanatomethyl-cyclohexan (=Isophorondiisocyanat oder IPDI), 2,4- und 2,6-Toluylendiisocyanat (TDI) sowie 4,4'-, 2,4'- und 2,2'-Diphenylmethandiisocyanat (MDI) geeignet. Als mindestens zwei Isocyanat-Gruppen aufweisendes Prepolymer sind insbesondere derartige Prepolymere, welche erhältlich aus mindestens einem der vorgenannten Polyisocyanate und mindestens einem Polyol sind. Als Polyole eignen sich insbesondere Polyoxyalkylenpolyole oder Polyesterpolyole mit mindestens zwei OH-Gruppen, insbesondere mit 2 oder mit 3 OH-Gruppen.

Durch Mischen der Amin-reaktive Substanz, welche mindestens zwei Amin-reaktive funktionelle Gruppen aufweist, mit der erfindungsgemässen Mannichbase tritt eine Reaktion der Aminischen Gruppen der Mannichbasen mit den Amin-reaktiven funktionellen Gruppen der Amin-reaktiven Substanz auf und es erfolgt eine Aushärtung.

Somit umfasst die vorliegende Erfindung auch eine zweikomponentige Zusammensetzung, welche aus einer ersten Komponente **K1** und einer zweiten Komponente **K2** besteht. Die erste Komponente **K1** umfasst mindestens einen Amin-reaktive Verbindung mit mindestens zwei funktionellen Gruppen, welche mit Aminen reagieren können. Die zweite Komponente **K2** umfasst mindestens eine Mannichbase, wie sie vorher bereits beschrieben wurde. Die als Amin-reaktive Verbindungen, welche mindestens zwei funktionelle Gruppen aufweisen, welche mit Aminen reagieren können, geeignete Verbindungen sind bereits vorgängig beschrieben worden.

Die erste Komponente **K1** umfasst vorteilhaft mehrere Amin-reaktive Verbindungen. So wird insbesondere die Verwendung von einem höher viskosen und einer niedrigviskosen Amin-reaktive Verbindung empfohlen. Als niedrigviskose Amin-reaktive Verbindung werden besonderes sogenannte Reaktivverdünner bevorzugt.

Die zweite Komponente **K2** kann neben der Mannichbase weitere Amine enthalten. Insbesondere handelt es sich hierbei um ein aliphatisches oder cycloaliphatisches Amin, bevorzugt Isophorondiamin (IPDA), enthält. Weiterhin bevorzugt kann die Komponente **K2** TETA oder TEPA enthalten. Dieses zusätzliche Amin kann bereits am Ende der Mannichbase oder erst beim Formulieren der Komponente **K2** beigefügt werden.

Beide Komponenten **K1** und **K2** können bei Bedarf weitere dem Fachmann bekannte Inhaltsstoffe umfassen. Derartige weitere Inhaltsstoffe sind insbesondere Füllstoffe, Weichmacher, Lösungsmittel, Katalysatoren und/oder Additive.

Als Füllstoffe gelten insbesondere Russe, Kreiden, insbesondere beschichtete Kreiden, Sande, Silikate, Leichtfüllstoffe, wie Keramik- oder Glaskugeln, insbesondere Keramik- oder Glashohlkugeln, pyrogene Kieselsäuren, Flugasche, als bevorzugt.

Als Lösungsmittel werden insbesondere derartige Lösungsmittel bevorzugt, welche nicht als VOC "volatile organic compounds" eingestuft werden. Besonders bevorzugt werden höher siedende Kohlenwasserstoffe.

Als Weichmacher sind insbesondere Phthalate und Adipate, insbesondere Düsodecylphthalat (DIDP) und Dioctyladipat (DOA).

Derartige zweikomponentige Zusammensetzungen lassen sich breit einsetzen. Insbesondere bevorzugt ist deren Verwendung als Klebstoff oder Dichtstoff, insbesondere als struktureller Klebstoff. Es hat sich nämlich gezeigt, dass die mittels den erfindungsgemässen Mannichbasen erzielbaren Eigenschaften insbesondere im Klebstoffbereich besonders erwünscht sind.

Insbesondere hat sich gezeigt, dass sich hohe Aushärtungsgeschwindigkeiten, insbesondere bei tiefen Temperaturen, erzielen lassen und dass hohe Glasübergangstemperaturen (Tg) erreicht werden können, auch wenn die Aushärtung kalt erfolgt, d.h. bei Raumtemperaturen. Dies ist besonders wichtig für Epoxidharz- Zusammensetzungen, da bisher für die Erzielung von hohen Tgs eingesetzte Mannichbasen-freie Aminhärter, zum Beispiel Isophorondiamin basierende Härter, entweder bei hohen Temperaturen, d.h. über 60°C, umgesetzt werden musste oder für welche nach der Raumtemperaturaushärtung eine Nachtemperung, d.h. nachfolgende Erwärmung auf Temperaturen von über 60°C, eingesetzt werden musste. Zudem tritt mit Mannichbasen-freie Aminhärtern umgesetzten Epoxydharzen vielfach das Problem auf, dass die Aushärtung im sogenannten Beta-Stadium stehen bleibt und die Endfestigkeit nur über ein nachfolgendes Erwärmen ermöglicht ist. Zudem liessen sich derartige Mannichbasen-freie Aminhärtern bei Temperaturen unter 10°C insbesondere unter 5°C nicht oder nur sehr schlecht aushärten. Diese Nachteile des Standes der Technik können durch erfindungsgemässe Mannichbasen behoben werden. Insbesondere lassen sich nach Aushärtung bei Raumtemperatur Glasumwandlungstemperaturen von über 80°C erreichen, ohne dass ein Nachtempern nötig ist. Zudem härten derartige Zusammensetzung auch bei tiefen Temperaturen, insbesondere unter 10°C, bevorzugt zwischen ―10°C und 5°C aus.

Für alle Anwendungen ist es, insbesondere aus ökotoxikologischen und arbeitshygenischen Aspekten, wichtig, dass mit den erfindungsgemässen Mannichbasen Härterkomponenten zur Verfügung gestellt werden können, welche frei von Phenolen, aber auch frei von anderen phenolischen Verbindungen, und bevorzugt auch frei, d.h. nicht mehr messbare Mengen enthaltend, von nicht umgesetzten Resorcin sind.

Nach dem Mischen der Komponenten **K1** und **K2** der beschriebenen zweikomponentigen Zusammensetzung wird der Klebstoff auf eine Substratoberfläche appliziert und mit einer weiteren Substartoberfläche gefügt. Die ausgehärtete Zusammensetzung wirkt als Klebschicht, welche befähigt ist, Kräfte zwischen den zwei Substratoberflächen des gebildeten Verbundkörpers zu übertragen.

Die zweikomponentige Zusammensetzung eignet sich aufgrund ihrer Eigenschaften speziell gut als struktureller Klebstoff im Hoch- und Tiefbau, sowie in der Industrie.

Beispielsweise kann eine derartige zweikomponentige Zusammensetzung, insbesondere eine zweikomponentige Epoxidharz-Zusammensetzung, d.h. wo die Komponente **K1** einen Diglycidylether umfasst, als Klebstoff für das Verkleben von faserverstärkten Composites eingesetzt werden. Ein illustrierendes Beispiel hierfür ist das Verkleben von Kohlenfaser-Lamellen beim Verstärken von Bauwerken, wie Brücken.

Weiterhin können erfindungsgemässe zweikomponentige Zusammensetzungen, insbesondere eine zweikomponentige Epoxidharz-Zusammensetzung, als Kunststoffmatrix für die Herstellung von faserverstärkten Composites eingesetzt werden. So lassen sich beispielsweise Kohlen- oder Glasfasern in eine zweikomponentige Zusammensetzung einbetten und können im ausgehärteten Zustand als Faser-Composite, beispielsweise in Form einer Lamelle, zum Einsatz kommen.

Ebenso können beispielsweise Fasergewebe oder -gelege mittels einer zweikomponentigen Zusammensetzung, insbesondere mittels einer zweikomponentigen Epoxidharz-Zusammensetzung, auf ein Bauwerk appliziert werden, und dort mit dem Bauwerk zusammen ein faserverstärktes Composite bilden.

### Beispiele

### Herstellung Mannichbasen

### a) mit Lösungsmittelverdünnung

1 Mol der in Tabelle 1 angegebenen phenolischen Verbindung wurde mit 3 Mol des in Tabelle 1 angegebenen Polyamins in technischer Qualität mit 90 g Methanol in einem Reaktionsgefäss unter Stickstoff vorgelegt und allenfalls bis maximal 80°C erwärmt, bis das Phenol in Lösung ging. Anschliessend wurde mittels Eisbad auf eine Temperatur zwischen 2 und 13 °C gekühlt. Unter Rühren wurde anschliessend 2 Mol Formaldehyd (als 37 % Formalin Lösung eingesetzt) unter intensivem Rühren und Eisbadkühlung zugetropft. Nachdem das Formaldehyd vollständig zugetropft wurde bei einem Druck von 400 mbar die Temperatur langsam auf 90 °C erhöht. Schliesslich wurde das Vakuum erhöht auf 50 mbar. Die erhaltene Menge Destillates entspricht der Menge der theoretischen Menge des Methanols und Wassers, welches eingesetzt und sich gebildet hat.

**Tabelle 1 Eigenschaften von Mannichbasen (Variation der phenolischen Verbindung). *Mittelwert einer Dreifachbestimmung **Nachweisgrenze unter 0.05 %**

| | ***M1*** | ***Ref. M1*** | ***Ref. M2*** |
|---|---|---|---|
| Phenolische Phenolische Verbindung | Resorcin | 3,5-Xylenol | m-Kresol |
| Qualität, Lieferant | distilled flakes, Clariant (Schweiz) | purum, Fluka (Schweiz) | ≥99%, Fluka (Schweiz) |
| Polyamin | TETA | TETA | TETA |
| Qualität, Lieferant | technisch, Fluka (Schweiz) | technisch, Fluka (Schweiz) | technisch, Fluka (Schweiz) |
| Restgehalt Polyamin* | 19.4 ± 1% | 31.3 ± 1.3% | 34.1 ± 0.3% |
| Restgehalt phenolische Verbindung* | 0% ** | 12.52 ± 1.3% | 15.11 ± 0.05 % |
| Viskosität [mPas] | 29'100 | 348 | 206 |
| Aminzahl [mg/gKOH] | 986 | 879 | 921 |

Die Viskosität wurde mittels Rheomat bei 20°C gemessen Kegel/Platte (40 mm Kegel, 300 Umdrehungen/s bzw. 20mm Kegel, 50 Umdrehungen/s).
Der Restgehalt des Polyamins wurde mittels GC/FID (Optima-5MS, 60 mg gelöst in 10 ml Ethylacetat, Trägergas He, externer 3 Punkt Kalibration im Konzentrationsbereich 2-6 mg/ml) und der Restgehalt der phenolischen Verbindung mittels HPLC/PDA (Varian, LiChrosphere 100 RP-18, Eluent Wasser, Acetonitil, UV 273 nm) bestimmt.
Die Aminzahl wurde titrimetrisch auf einem Memotritator DL-55 der Firma Mettler, Schweiz bestimmt.

### b) ohne Lösungsmittelverdünnung

1 Mol Resorcin wurde mit 1.7 Mol des in Tabelle 2 angegebenen Polyamins in technischer Qualität in einem Reaktionsgefäss unter Stickstoff vorgelegt und auf 140°C erwärmt, bis kein festes Resorcin mehr vorhanden war. Anschliessend wurde mittels Eisbad auf eine Temperatur zwischen 90°C und 80°C gekühlt, nochmals 1.7 Mol des in Tabelle 2 angegebenen Polyamins in technischer Qualität zugegeben und auf eine Temperatur zwischen 5°C und 10°C gekühlt. Unter Rühren wurde anschliessend 2 Mol Formaldehyd (als 37 % Formalin Lösung eingesetzt) unter intensivem Rühren und Eisbadkühlung zugetropft. Nachdem das Formaldehyd vollständig zugetropft war bei einem Druck von 400 mbar die Temperatur langsam auf 150°C erhöht. Schliesslich wurde das Vakuum erhöht auf 50 mbar. Die erhaltene Menge Destillates entspricht der Menge der theoretischen Menge des Wassers, welches eingesetzt und sich gebildet hat. Es resultierte bei ***M2*** und ***M3*** jeweils eine stabile Mannichbase, während die Referenzbeispiele mit IPDA (***Ref. M3***), Diethylentriamin (DETA) (***Ref. M4***) als Polyamin gelierten.

**Tabelle 2 Mannichbasen Variation des Polyamins.**

| | ***M2*** | ***M3*** | ***Ref. M3*** | ***Ref. M4*** |
|---|---|---|---|---|
| Phenolische Verbindung | Resorcin | Resorcin | Resorcin | Resorcin |
| Qualität, Lieferant | distilled flakes, Clariant (Schweiz) | distilled flakes, Clariant (Schweiz) | distilled flakes, Clariant (Schweiz) | distilled flakes, Clariant (Schweiz) |
| Polyamin | TETA | TEPA | IPDA | DETA |
| Qualität, Lieferant | technisch, Fluka (Schweiz) | technisch, Fluka (Schweiz) | puriss, Fluka (Schweiz) | ≥98%, Fluka (Schweiz) |

### Verwendung als Härter

Die Mannichbasen wurden als Härterkomponente **K2** für eine Epoxydharzkomponente **K1** eingesetzt. Die Epoxydharzkomponente bestand entweder aus 80 Gew.-% Diglycidylether von Bisphenol-A (Araldite® GY 250, Huntsman) und 20 Gew.-% Hexandioldiglycidylether (Araldite® DY-H, Huntsman, Epoxyzahl 6.25 - 6.65) **(K1-1)** oder aus 85 Gew.-% Diglycidylether von Bisphenol-A (Araldite® GY 250, Huntsman) und 15 Gew.-% Trimethylolpropantriglycidylether (Araldite® DY - T / CH, Huntsman) **(K1-2).**

**Tabelle 3. Eigenschaften ausgehärteter Zusammensetzungen.^{†}n.b.=nicht bestimmt.**

| | ***Z1*** | ***Ref.Z1*** | ***Ref.Z2*** | ***Z2*** |
|---|---|---|---|---|
| **K1** | ***K1-2*** | ***K1-2*** | ***K1-2*** | ***K1-1*** |
| **K2** | ***M1*** | ***Ref.1*** | ***Ref.2*** | *M3* |
| **K1/K2** [g/g] | 80/20 | 80/20 | 80/20 | 80/20 |
| Topfzeit [min] | 18 | 22 | 20 | 29 |
| ZF(1d)[MPa] | 43.6 | 47.1 | 51.6 | n.b.^{†} |
| BD (1d) [%] | 1.4 | 1.6 | 1.8 | n.b.^{†} |
| Tg [°C] | 96 | 108 | 105 | n.b.^{†} |

Die Topfzeit wurde bestimmt, indem 100 g der gemischten Komponenten bei Raumtemperatur in einem Becher mittels Spatel gerührt wurde. Als Topfzeit wurde diejenige Zeit angegeben, bei welcher der Ansatz gelierte.
Die Zugfestigkeit (ZF (1d)) und die Bruchdehnung (BD(1D)) wurden nach 1 Tag Aushärtung bei Raumtemperatur nach ISO 527 mit einer Messgeschwindigkeit von 5 mm/min auf einer Zwick-Zugfestigkeitsapparatur gemessen.
Die Glasumwandlungstemperatur wurde als Peakmaximum mittels DSC (0-250°C 10°/min) gemessen.

### Verwendung als Klebstoff

Mit den Zusammensetzungen **Z1** und **Z2** wurden Aluminiumplatten sowie Stahlplatten verklebt. Die Klebstoffe zeigten gute Haftung und gute Verbundfestigkeiten.

Weiterhin wurde die Mannichbase **M1** als Härterkomponente **K2** mit der A-Komponente des Sikadur®-30 (kommerziell erhältlich bei Sika Schweiz AG), welche auf Bisphenol-A-Diglycidylether und einem Epoxy-Reaktivverdünner basiert, als Komponente **K1** im Mischungsverhältnis 1:10 gemischt und eine Beton-Platte mit einer Betondecke verklebt. Die Verklebung wies eine gute Haftung sowie eine gute Verbundfestigkeit auf.

## Patentansprüche

1. Mannichbase, welche aus Resorcin, Formaldehyd sowie Triethylentetramin und/oder Tetraethylenpentamin herstellbar sind.

2. Mannichbase gemäss Anspruch 1, **dadurch gekennzeichnet, dass** die Molverhältnisse Resorcin : Formaldehyd: (Triethylentetramin + Tetraethylenpentamin) = 1 : 1.5-2.5 : 2.5-3.5, insbesondere 1: etwa 2 : etwa 3, betragen.

3. Mannichbase gemäss Anspruch 1 oder 2, **dadurch gekennzeichnet, dass** das Triethylentetramin und/oder Tetraethylenpentamin ein Triethylentetramin und/oder Tetraethylenpentamin in technischer Qualität ist.

4. Mannichbase gemäss einem der vorhergehenden Ansprüche, **dadurch gekennzeichnet, dass** die Aminzahl zwischen 800 und 1100 mg/g KOH, insbesondere im Bereich zwischen 900 und 1000 mg/g KOH, bevorzugt im Bereich zwischen 950 und 1000 mg/g KOH, ist.

5. Mannichbase gemäss einem der vorhergehenden Ansprüche, **dadurch gekennzeichnet, dass** die Mannichbase keine messbaren Mengen an nicht umgesetzten Resorcin aufweist.

6. Verfahren zur Herstellung einer Mannichbase gemäss einem der Ansprüche 1 bis 5, **dadurch gekennzeichnet, dass** Resorcin, Triethylentetramin und/oder Tetraethylenpentamin mit Formaldehyd bei einer Temperatur von unter 25°C zur Reaktion gebracht werden.

7. Verfahren gemäss Anspruch 6, **dadurch gekennzeichnet, dass** die Reaktion ohne Anwesenheit von zusätzlichen tertiären Aminen, welche nicht bereits in technischem Triethylentetramin und/oder technischem Tetraethylenpentamin vorhanden sind, erfolgt.

8. Verfahren gemäss Anspruch 6 oder 7, **dadurch gekennzeichnet, dass** Formaldehyd zu einer Vormischung umfassend Resorcin und Triethylentetramin und/oder Tetraethylenpentamin unter Rühren zugegeben wird.

9. Verfahren gemäss einem der Ansprüche 6 bis 8, **dadurch gekennzeichnet, dass** ein Lösungsmittel, insbesondere eine Alkohol, bevorzugt Methanol, vor der Reaktion mit Formaldehyd zugegeben wird.

10. Verfahren gemäss einem der Ansprüche 6 bis 9, **dadurch gekennzeichnet, dass** nach der Reaktion mit Formaldehyd Wasser und gegebenenfalls Lösungsmittel abdestilliert wird.

11. Zweikomponentige Zusammensetzung bestehend aus einer ersten Komponente **K1,** welche mindestens einen Amin-reaktive Verbindung mit mindestens zwei funktionellen Gruppen, welche mit Aminen reagieren können, umfasst, und einer zweiten Komponente K2, welche mindestens eine Mannichbase gemäss einem der Ansprüche 1 bis 5 umfasst.

12. Zweikomponentige Zusammensetzung gemäss Anspruch 11, **dadurch gekennzeichnet, dass** die erste Komponente K1 neben der Mannichbase gemäss einem der Ansprüche 1 bis 5, ein weiteres Amin, insbesondere ein aliphatisches oder cycloaliphatisches Amin, bevorzugt Isophorondiamin, enthält.

13. Zweikomponentige Zusammensetzung gemäss Anspruch 11 oder 12, **dadurch gekennzeichnet, dass** die Amin-reaktive Verbindung mit mindestens zwei funktionellen Gruppen, welche mit Aminen reagieren können, in der Komponente K1 ein Diglycidylether, insbesondere ein Diglycidylether von Bisphenol-A, Bisphenol-F oder Bisphenol-A/F, ist.

14. Zweikomponentige Zusammensetzung gemäss Anspruch 11 oder 12, **dadurch gekennzeichnet, dass** die Amin-reaktive Verbindung mit mindestens zwei funktionellen Gruppen, welche mit Aminen reagieren können, in der Komponente K1 ein Polyisocyanat oder ein mindestens zwei Isocyanat-Gruppen aufweisendes Prepolymer ist.

15. Verwendung der zweikomponentigen Zusammensetzung gemäss einem der Ansprüche 11 bis 14 als Klebstoff oder Dichtstoff, insbesondere als struktureller Klebstoff.

16. Ausgehärtete Zusammensetzung **dadurch gekennzeichnet, dass** sie durch Mischen und Aushärten der zwei Komponenten K1 und K2 einer zweikomponentigen Zusammensetzung gemäss einem der Ansprüche 11 bis 14 erhalten wird.

17. Verbundkörper, welcher durch eine ausgehärtete Zusammensetzung gemäss Anspruch 16 als Klebschicht aufweist.

18. Verwendung einer Mannichbase gemäss einem der Ansprüche 1 bis 5 als Härter für eine Amin-reaktive Substanz, welche mindestens zwei Amin-reaktive funktionelle Gruppen aufweist.

19. Verwendung gemäss Anspruch 18, **dadurch gekennzeichnet, dass** die Amin-reaktive Substanz, welche mindestens zwei Amin-reaktive funktionelle Gruppen aufweist, ein Diglycidylether, insbesondere ein Diglycidylether von Bisphenol-A, Bisphenol-F oder Bisphenol-A/F, ist.

20. Verwendung gemäss Anspruch 18, **dadurch gekennzeichnet, dass** die Amin-reaktive Substanz, welche mindestens zwei Amin-reaktive funktionelle Gruppen aufweist, ein Polyisocyanat oder ein mindestens zwei Isocyanat-Gruppen aufweisendes Prepolymer ist.
